# EUROPEAN PATENT APPLICATION

(11) **EP 4 848 302 A1**
(43) Date of publication of application: **02.12.2026**
(21) Application number: 26176416.1
(22) Date of filing: 05.05.2026
(51) Int. Cl.: G16H 40/63, A61B 5/08, G16H 40/40

(54) **SYSTEMS AND METHODS FOR MEDICAL DEVICE ACCESSORIES**

(30) Priority: 27.05.2025 US 202519219919
(71) Applicant: GE Precision Healthcare LLC, Waukesha, WI 53188 (US)
(72) Inventor: NYBLOM, Henna, 00510 Helsinki (FI); SERMI, Tommi, 00510 Helsinki (FI)
(74) Representative: Kilburn & Strode LLP

(57) **Abstract**

Methods and systems for gas analyzer systems are herein presented. For example, a gas analyzer system (200) comprises a gas analyzer (102, 7) having a processor (104) and a reading system (116); and at least one medical accessory (120, 24, 27) comprising a data storage system (122), wherein the processor is configured to: establish a communicative connection with the data storage system of the medical accessory (402); in response to establishing the communicative connection, receive accessory specific data of the medical accessory by the reading system from the data storage system (406); determine compatibility of the medical accessory and the gas analyzer based on the accessory specific data; determine a remaining useful lifetime of the medical accessory based on the accessory specific data (408); when the medical accessory and the gas analyzer are compatible, operate the gas analyzer based on the accessory specific data (510); and when the medical accessory and the gas analyzer are incompatible, generate a notification of incompatibility (512).

## Description

### FIELD

Embodiments of the subject matter disclosed herein relate to medical device accessories, and more particularly, to systems and methods for determining compatibility and performance status of one or more medical device accessories for a gas analyzer system.

### BACKGROUND

In anesthesia and intensive care, the condition of the patient is often monitored by analyzing the gas inhaled and exhaled by the patient for its content. For this reason, either a small portion of the respiratory gas is delivered to a gas analyzer or the gas analyzer is directly connected to the respiratory circuit. Such gas analyzers often are equipped with one or more exchangeable accessories, including water traps and sample lines. These accessories have useful lifetimes, after which point the medical accessory's performance may decrease.

### BRIEF DESCRIPTION

In one example, a gas analyzer system comprises a gas analyzer comprising a reading system and one or more processors configured to execute instructions stored in memory; and at least one medical accessory comprising a data storage system, wherein the one or more processors are configured to: establish a communicative connection with the data storage system of the at least one medical accessory; in response to establishing the communicative connection, receive accessory specific data of the at least one medical accessory by the reading system from the data storage system; determine compatibility of the at least one medical accessory and the gas analyzer based on the accessory specific data; determine a remaining useful lifetime of the at least one medical accessory based on the accessory specific data; when the at least one medical accessory and the gas analyzer are compatible, operate the gas analyzer based on the accessory specific data; and when the at least one medical accessory and the gas analyzer are incompatible, generate a notification of incompatibility.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The present disclosure will be better understood from reading the following description of non-limiting embodiments, with reference to the attached drawings, wherein:
FIG. 1 shows a block diagram of a system comprising medical host device and at least one medical accessory, in accordance with one or more embodiments of the present disclosure;
FIG. 2 shows a gas analyzer connected to a ventilation circuit ventilating a patient, in accordance with one or more embodiments of the present disclosure;
FIG. 3 shows a medical gas analyzer, in accordance with one or more embodiments of the present disclosure;
FIG. 4 shows a flowchart illustrating a high-level method for the system of FIG. 1, in accordance with one or more embodiments of the present disclosure;
FIG. 5 shows a flowchart illustrating a method for determining compatibility between the medical accessory and the medical host device, in accordance with one or more embodiments of the present disclosure;
FIG. 6 shows a flowchart illustrating a method for determining compatibility between a first medical accessory and a second medical accessory connected to the medical host device, in accordance with one or more embodiments of the present disclosure;
FIG. 7 shows a flowchart illustrating a method for determining a remaining useful lifetime of the medical accessory connected to the medical host device, in accordance with one or more embodiments of the present disclosure;
FIG. 8 shows a flowchart illustrating a method for determining an operational status of the medical accessory connected to the medical host device, in accordance with one or more embodiments of the present disclosure;
FIG. 9 shows a flowchart illustrating a method for determining an operational mode of a host device based on accessory specific data received from the connected medical accessory;
FIG. 10 shows a graph of measured flow resistances; and
FIG. 11 shows a flowchart illustrating a method for determining quality of a communicative connection between a medical accessory and a host device.

### DETAILED DESCRIPTION

The following description relates to various embodiments of methods and systems for medical device accessories. More particularly, systems and methods are herein provided for determining compatibility between medical accessories and a host device, determining host device parameters based on data from a connected medical accessory, and estimating a remaining useful lifetime of a connected medical accessory.

In hospital settings, for example in operative and intensive care settings, medical devices such as gas analyzers typically incorporate one or more medical accessories. These medical accessories, such as water traps and sample lines in the case of gas analyzers, are generally exchangeable. Due to manufacturing processes, slight variations in the features of medical accessories can occur. For example, different brands of accessories may have slightly different features and/or medical accessories of the same brand may have different features based on variations in manufacturing. Without considering these variations, all medical accessories of a type may be treated the same, leading to faster aging and more frequent replacement demands.

Each medical accessory may have a useful lifetime. Currently, a recommended useful lifetime is provided as a label on the medical accessory itself and a manual method of marking the medical accessory with an expiration date and checking the date with every use is used to determine when the medical accessory demands exchanging. This manual method is prone to user error, including when clinicians and/or technicians forget to check, may result in medical accessories being used longer than their useful lifetime, which can result in medical accessory malfunctions, issues for the host device, and medical accessory degradation that affects the medical accessory's performance. Determining a remaining useful lifetime of an accessory in a clinical setting may be difficult due to factors such as liquids and nebulized drugs which can quickly shorten a remaining useful lifetime. Such an event may cause the medical accessory to abruptly stop functioning without warning, reducing the real time efficacy of the host device.

Further still, some medical accessories may be incompatible with a particular host device or with another accessory connected to the host device. Currently, a manual process of analyzing compatibility between medical accessories and devices, however this step takes extra time and is prone to user error.

Methods and systems are herein provided for a medical device and accessory system that at least partially addresses the issues outlined above. Medical device accessories may be equipped with a memory storage device, such as a near field communication (NFC) tag or radio frequency identification (RFID) tag, that is configured to transmit data wirelessly. The memory storage device may store accessory specific data, such as configuration specifics (e.g., as determined from product specific measurement information, an identifier (e.g., a UID, identification number, etc.), or the like), a batch, relevant dates (e.g., commissioning date), usage history, operational states, and expected model of a compatible host.

This accessory specific data may be transmitted to a host device to which the medical accessory is connected. The host device may use the accessory specific data to determine compatibility of the medical accessory with the host device, as well as compatibility of the medical accessory with another connected accessory in some examples. The host device may also use the accessory specific data to determine its own operational modes. Further, the host device may determine a remaining useful lifetime of the medical accessory based on the accessory specific data and measured data, like differential pressure, flow, and resistance of the accessory, and detect operational statuses of the medical accessory, including malfunctions, when the medical accessory is connected. In some examples, when a malfunction or a contamination is detected, one or more operational states of the host device may be altered. In this way, the currently manual processes of determination of remaining useful lifetime as well as compatibility may be mitigated by an automated process that is more accurate and reduces user time.

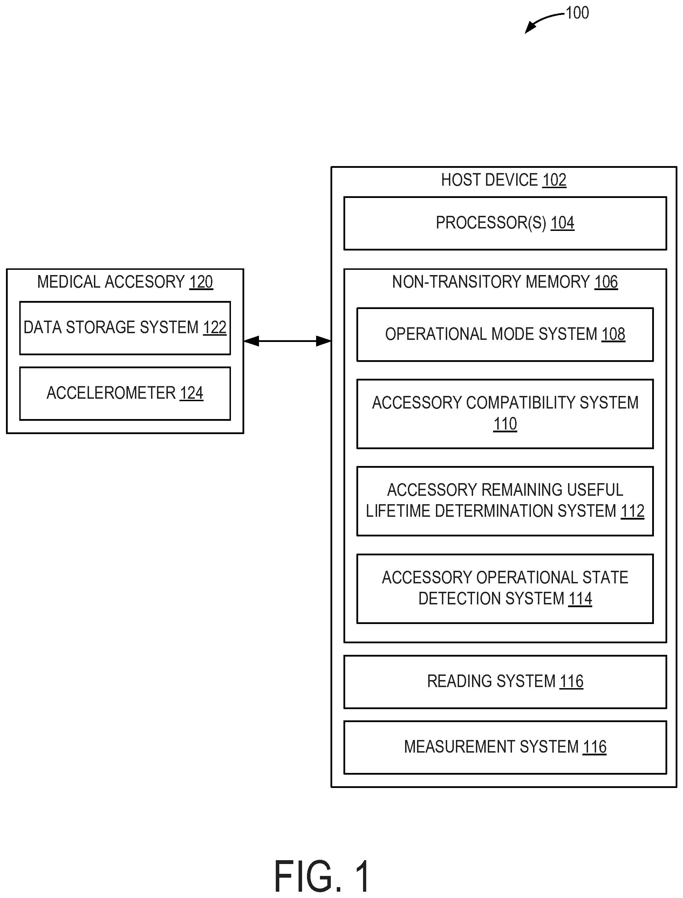

FIG. 1 shows a system 100 comprising a host device 102 and one or more medical accessories 120, wherein each of the one or more medical accessories 120 comprise a data storage system 122. The host device 102 may be a gas analyzer device in one example, though it should be appreciated that the systems and methods herein described may be applicable to other devices such as ventilators using CO₂ absorbers and expiratory valves. In examples when the host device 102 is a gas analyzer device, the one or more medical accessories 120 may comprise a water trap and/or a sample line.

As noted, each medical accessory 120 may comprise a data storage system 122. The data storage system 122 may be an electrical wireless transmitting memory device (e.g., a memory device), such as an NFC tag or RFID tag. The data storage system 122 may store accessory specific data, including physical properties of the medical accessory, operational properties of the medical accessory, and functional properties of the medical accessory. Examples of information stored in the data storage system 122 may include length of sample line, diameter of sample line, drying capabilities of sample line, intended sample line flow and vacuum, sample line type and material, model and mode (e.g., neonatal, pediatric, adult), material type and resistance of film/membrane, presence of connecting pieces (e.g., cup vs no cup), commissioning date, hours in use in anesthetic/non-anesthetic and mode (e.g., adult, pediatric, neonatal), number of previous hosts, number of occlusion events, number and type of nebulization events, bacterial and viral filtration efficiency, expected lifetime, remaining useful lifetime, and expected model of compatible host. In some examples, the data storage system 122 may be a rewritable memory tag, thus allowing the data stored therein to be updated. In this way, usage history of the device may be kept up to date as the medical accessory ages.

In some examples, the medical accessory 120 may additionally comprise an accelerometer 124, or other type of inertial measurement unit. The accelerometer 124 may detect a relative orientation of the medical accessory 120. Data from the accelerometer may be included in usage history as stored in the data storage system 122.

The host device 102 may comprise one or more processors 104 and non-transitory memory 106. The one or more processors 104 may be configured to execute machine readable instructions stored in non-transitory memory 106. Processor(s) 104 may be single core or multicore, and the programs executed thereon may be configured for parallel or distributed processing. In some embodiments, the processor(s) 104 may optionally include individual components distributed throughout two or more devices, which may be remotely located and/or configured for coordinated processing. In some embodiments, one or more aspects of the processor(s) 104 may be virtualized and executed by remotely-accessible networked computing devices configured in a cloud computing configuration.

The host device 102 may further comprise a reading system 116. The reading system 116 may be configured to receive transmitted data from one or more memory storage devices, such as data storage system 122 of the medical accessory 120. For example, the host device 102 may be equipped with a scanner/receiver (e.g., a reader) configured to receive accessory specific data from the data storage system 122 of each connected device. The reader of the reading system 116 may be configured with a specific scanning field, whereby the reader can establish a communicative connection with and receive data from memory storage devices that are within range of the scanning field. In some examples, the reading system 116 may be further configured to determine a field strength between the host device 102 and a connected medical accessory, either by inference or by direct measurement (e.g., via a near-field probe used with a spectrum analyzer or field meter for measuring magnetic field strength).

In one example, the host device 102 may comprise a coupling mechanism for one or more medical accessories that may be connected to the host device 102. As a non-limiting example, the host device 102 may be a gas analyzer that includes a coupling mechanism (e.g., an inlet port) for mechanically and pneumatically coupling a water trap to the gas analyzer. For example, the coupling mechanism may comprise standardized connectors like snap-in or Bayonet-style connectors, Luer locks or slips, or the like, in some examples including components like o-rings or other sealing components. For example, the water trap may comprise tabs or latches that snap into place or twist-lock into a recessed housing (e.g., the inlet port) on the analyzer. The water trap itself may comprise a second coupling mechanism (e.g., an inlet port) for mechanically and pneumatically coupling a sampling line to the water trap. For example, the second coupling mechanism may comprise barbed fittings, Luer lock/slip connectors, molded connectors, push-in fittings, or the like. Thus, the sampling line may be coupled to (e.g., pushed onto or into), the second coupling mechanism (e.g., the inlet port) of the water trap.

When the one or more medical accessories are positioned within the given inlet ports of the gas analyzer or other medical accessory, their memory storage devices (e.g., data storage system 122) may be within range of the reading system 114 of the host device 102. Thus, when positioned according to the coupling mechanisms described above. The communicative connection between the accessory and the host device may be established. Further, a quality of the communicative connection may be assessed based on the determined field strength of the communicative connection, which may inform for possible misalignment or malpositioning.

Non-transitory memory 106 may store an operational mode system 108, an accessory compatibility system 110, an accessory remaining useful lifetime determination system 112, and an accessory operational state determination system 114. The operational mode system 108 may store instructions executable by the one or more processor(s) 104 to determine an operational mode for the host device 102. As an example, the operational mode system 108 may determine a currently set operational mode of the host device 102, for example as set by a user, and determine an intended operational mode for the host device 102 based on the accessory specific data received from the medical accessory 120. As an example, the operational mode system 108 may determine a currently set operational mode as "adult". The accessory specific data received from the medical accessory 120 may indicate that the medical accessory 120 is a neonatal accessory. The operational mode system 108 may then determine whether to switch the host device 102 operational mode to "neonatal", for example based on operational modes of other connected accessories and/or other user-defined parameters.

The medical accessory compatibility system 110 may be configured to determine accessory-to-host device compatibility for connected accessories. In some examples, the medical accessory compatibility system 110 may also be configured to determine accessory-to-accessory compatibility for connected accessories. Accessory-to-host device compatibility and/or accessory-to-accessory compatibility may be determined based on the accessory specific data of the respective connected accessories.

The medical accessory remaining useful lifetime determination system 112 may be configured to determine, for the connected medical accessory 120, a remaining useful lifetime. The medical accessory remaining useful lifetime determination system 112 may be configured to determine the remaining useful lifetime based on the accessory specific data received from the data storage system 122 and in some examples measured values. As an example, the accessory specific data received from the data storage system 122 may comprise data of physical properties of the medical accessory 120, operational properties of the medical accessory 120, functional properties of the medical accessory 120, accessory history (e.g., previous treatment operations, number of occlusion events, etc.). Other data, such as host properties, such as gas analyzer assembly and configuration and host mode, and properties of other connected accessories may also be considered when determining the remaining useful lifetime. Further, the medical accessory remaining useful lifetime determination system 112 may utilize one or more values measured during use, such as flow, differential pressure, and resistance, as determined by measurement system 118 described below, in determining the remaining useful lifetime. The medical accessory remaining useful lifetime determination system 112 may comprise one or more of machine learning model(s), mathematical algorithms, and the like to determine the remaining useful lifetime based on these properties and measured values.

The medical accessory operational state determination system 114 may be configured to determine a current operational state of the medical accessory 120. The operational state of the medical accessory 120 may be, as non-limiting examples, operating as expected, malfunctioning, or the like. For example, certain events may cause the medical accessory 120 to malfunction, such as a puncture to a water trap membrane. Parameters of the medical accessory 120 may be monitored in order to determine the current operational state, as will be further described below.

The host device 102 may additionally comprise a measurement system 118. The measurement system 118 may comprise one or more sensors configured to measure accessory values such as flow, differential pressure, and resistance. For example, the measurement system 118 may include a flow sensor, such as a thermal mass flow sensor or differential pressure-based flow meter, configured to measure amount of gas per unit time, and a differential pressure sensor configured to compare the pressure between an inlet of the sampling line and an outlet of the sampling line that leads into the host device. Resistance may be determined as a function of the differential pressure (e.g., Δ*P*) and flow (e.g., as a ratio or quotient of differential pressure to flow). The measurement system 118 may be configured to iteratively and/or near-continuously measure these values as the host device 102 operates. For example, flow, differential pressure, and resistance may be measured at a frequency of 50 to 200 Hz. Flow may be measured to monitor sampling flow/rate and in order to detect sudden drops in flow or sampling rate, as per the accessory operational state detection system 114. For example, measured values may be temporarily or permanently stored in non-transitory memory 106 and accessed as per the accessory operational state detection system 114 for detection of accessory current operational state. Further, in some examples, the measured values may be displayed visually in a graph output to a display device communicatively coupled to the host device for user visualization.

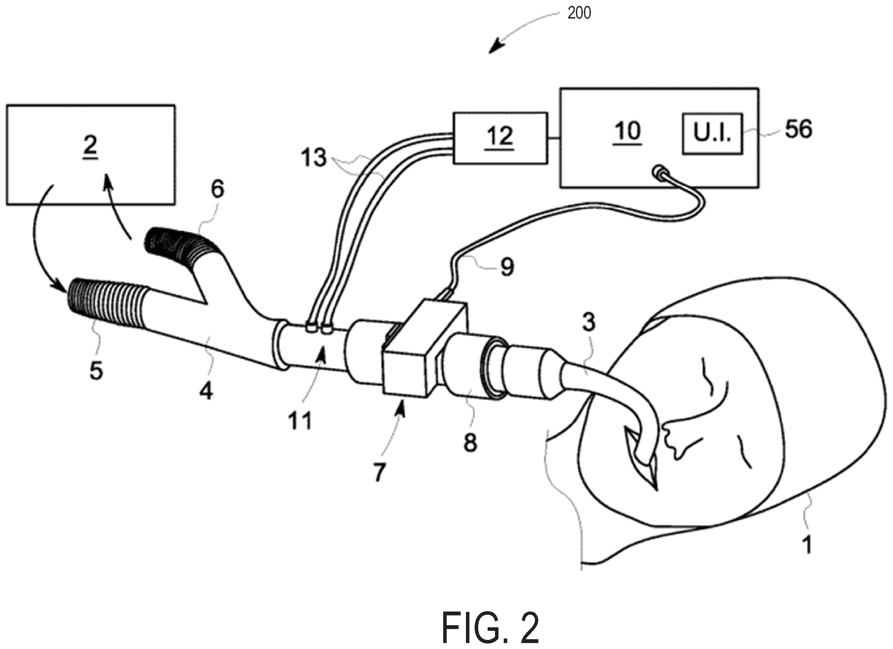

Turning to FIG. 2, a gas analyzer system 200 is shown. The gas analyzer system 200 may be an example of the host device 102 described with respect to FIG. 1. The gas analyzer system 200 may be included as part of a respiratory circuit. In the example shown in FIG. 2, a patient 1 is connected to a ventilator 2 using an intubation tube 3, a Y-piece 4, an inspiratory limb 5, and an expiratory limb 6. A gas analyzer 7 which may comprise an adapter 8 is connected to the intubation tube. The gas analyzer 7 in FIG. 2 is a mainstream gas analyzer measuring gases flowing between the ventilator 2 and the patient 1 without withdrawing samples of the gas to a separate gas analyzer. In other embodiments, such as a side stream gas analyzer, the gas analyzer may be positioned at a distance from the flow between the ventilator 2 and the patient 1.

The gas analyzer shown in FIG. 2 may be electrically connected via a cable 9 to the patient monitor 10. The gas component measured may be carbon dioxide (CO₂), nitrous oxide (N₂O), or any of the volatile anesthetic agents - e.g., halothane, enflurane, isoflurane, desflurane, and sevoflurane. Additionally, there may be a spirometry adaptor 11 for measuring the gas flow in the respiratory circuit. In this example, the sensor 12 is located at the distal end of two pressure relying tubes 13. The spirometry sensor may be separately connected as in FIG. 1 or it can be integrated into the mainstream gas analyzer, and may be similarly provided and incorporated in side stream gas analyzer.

The gas analyzer system 200 may be coupled to a patient monitor 10 that includes a user interface 56. Outputs from the sensor 12 may be displayed via the user interface 56 on the patient monitor 10, allowing clinicians and/or technicians to view and analyze sensor outputs as they are generated, for example during a surgical procedure or ICU monitoring.

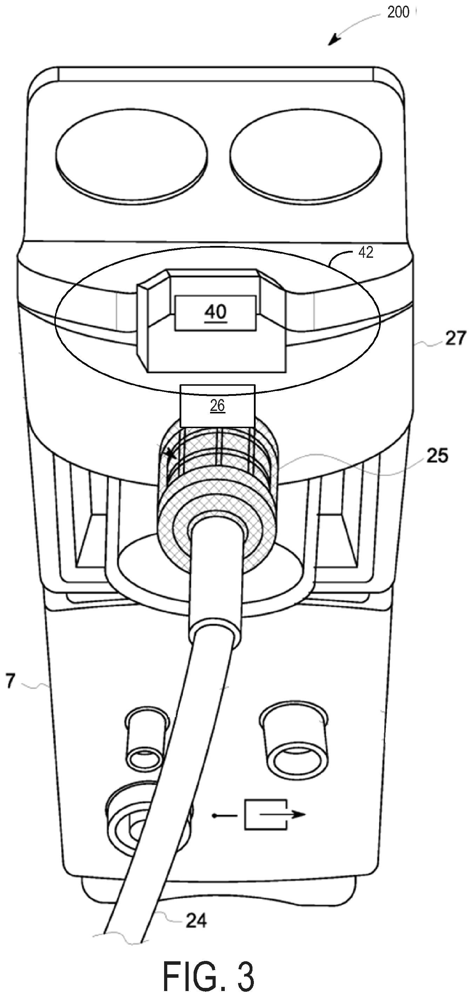

In FIG. 3, shows an example of the gas analyzer 7. In particular, the example shown in FIG. 3 is a side stream gas analyzer. In the example, the gas analyzer 7 is connected to the ventilation circuit by sampling line 24. The sampling line 24 comprise a connector 25 configured to couple the sampling line 24 to the gas analyzer 7. The connector 25 may be configured to mate with a port of the gas analyzer 7 or water trap 27. The gas analyzer 7 additionally comprises a sampling pump configured to actively draw gas at a fixed rate (e.g., 50-250 mL/min) to the analyzer.

The water trap 27 may be coupled to the gas analyzer 7 at a coupling port of the gas analyzer 7. The water trap 27 may be configured to trap liquids that form in the sampling line 24, thereby mitigating humidity and liquids from interfering with gas measurements by the gas analyzer 7.

As described herein, the sampling line 24 and the water trap 27 may each be equipped with a memory device capable of transmitting accessory specific data thereof to the gas analyzer 7. The memory devices may be tags, e.g., NFC tags, that are arranged at a particular position of the respective medical accessory. The gas analyzer 7 may comprise a reader device 40, as described above. The reader device may be positioned to connect with a corresponding memory device of a coupled medical accessory. As an example, an NFC tag 26 of the sampling line 24 may be incorporated at a side of the connector 25, in a flange wing, connector lock ring, or the like. While not depicted in FIG. 3, the water trap 27 may also comprise a memory device. The reader device 40 may be arranged such that a range of a field 42 thereof overlaps with the memory device(s) of the medical accessor(ies), e.g., with the NFC tag 26, when the medical accessor(ies) are positioned and oriented in a desired configuration. A communicative connection between the NFC tag of the sampling line, and/or the memory device of the water trap, and the gas analyzer may be established when the connector 25 is oriented with the NFC tag positioned at least partially within the field 42 of the reader device 40. This may reduce the possibility of the reader receiving data from medical accessories that are not connected to the host device or do not comprise a memory device in the proper position (e.g., an improperly manufactured accessory).

As a non-limiting example, the reader device 40 may be positioned vertically above a connecting port configured to couple to the water trap 27 and a connecting port of the water trap 27 configured to couple to the connector 25 of the sampling line 24. Using the sampling line 24 as an example, the NFC tag 26 may be oriented towards a vertical top of the gas analyzer 7 when the connector 25 is coupled to the corresponding connecting port of the gas analyzer 7 in order to be within range of the reader device 40 (e.g., with the field 42). When the NFC tag 26 is positioned in other orientations, such as towards a vertical bottom of the gas analyzer 7, the NFC tag 26 may not be in range of the reader device 40 and thus no communicative connection between the gas analyzer 7 and the sampling line 24 may be established. It should be understood that these relative positions and orientations as described are merely exemplary and other arrangements are possible.

In some examples, the memory device of the medical accessory and/or the reader device of the host device may be configured with relatively short fields. For example, the memory device and the reader device may be equipped with NFC technology. In one example, the field of the reader device may have a radius ranging between 1 and 5 cm, such as 4 cm. In contrast, an RFID tag may have a range with a radius between 10 cm and 10 meters. A smaller radius of range may ensure that the reader device does not erroneously connect to medical accessories not coupled to the host device (e.g., medical accessories stored in the same room as the host device) or to medical accessories with improperly positioned memory tags. In some examples, once a communicative connection between an accessory and the gas analyzer is made, by way of the memory device of the medical accessory being within range of the field of the reader device, a quality of the communicative connection and/or misalignments in connection may be determined, as will be further described below.

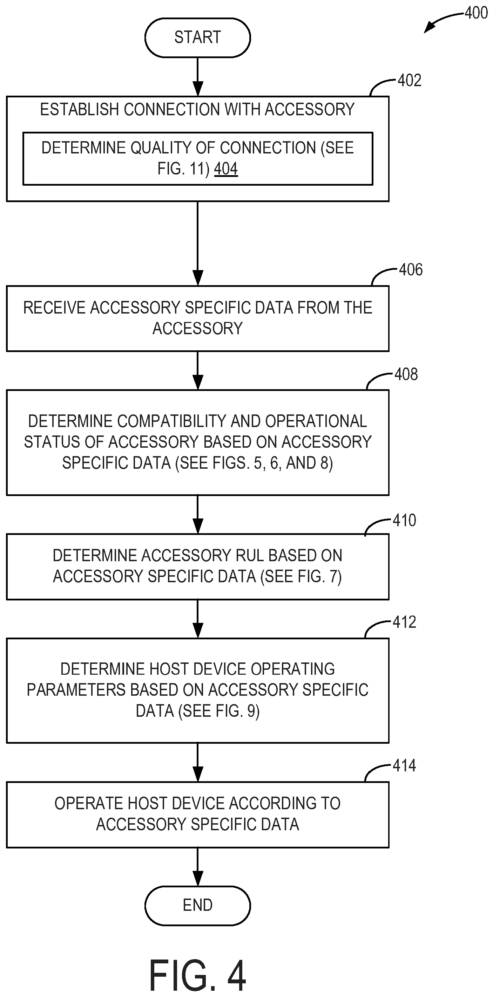

Turning now to FIG. 4, a flowchart illustrating a high-level method 400 for a system, such as system 100 described with respect to FIG. 1. As described above, the system may comprise a host device (e.g., host device 102) and one or more medical accessories (e.g., medical accessory 120). The method 400 herein described may be executed by a host device. In particular, the method 400 may be executed by one or more processors according to instructions stored in memory of the host device, e.g., by processor(s) 104 according to instructions stored in memory 106). The host device as herein described may be a gas analyzer and the one or more medical accessories may be one or more of a water trap and a sample line. In other examples, the host device may be a different type of device.

At 402, method 400 includes establishing a communicative connection with a medical accessory. As described herein, the medical accessory may comprise a data storage device, such as a wireless transmitting memory tag. The host device may establish the communicative connection between the host device and the medical accessory when the data storage device of the medical accessory is positioned and oriented to allow a field of a reader device of the host device to recognize it. For example, the reader device of the host device may be fixedly positioned with respect to connecting ports of the host device that are configured to receive the accessories. The communicative connection between the host device and the medical accessory may be established when the data storage device (e.g., the memory tag) is within range of the reader device. In order for its data storage device to be within range of the reader device, the medical accessory may be oriented in a particular fashion. For example, as shown in FIG. 3, the data storage device may be oriented with at a vertical top region in examples when the reader device is positioned vertically above the connecting port of the medical accessory. When the medical accessory is positioned with other orientations, such as with the data storage device positioned at a vertical bottom, the data storage device may not be within range of the reader device of the host device and thus no communicative connection between the host device and the medical accessory may be established. The host device may demand communicative connection to the medical accessory for operation to occur, in some examples. For example, the host device may not operate until communicative connections are established to a water trap and a sampling line.

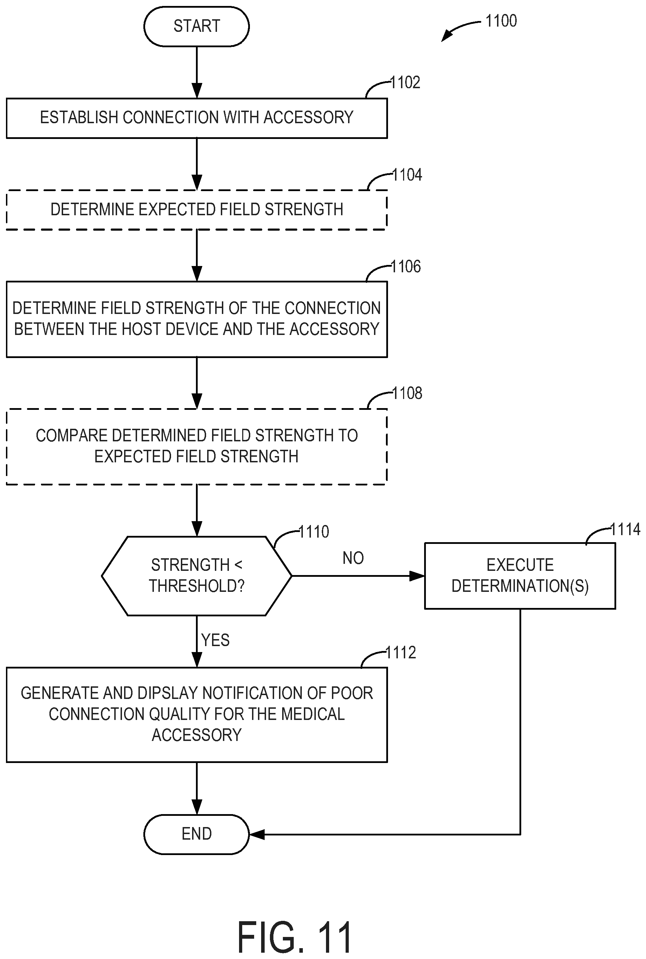

In some examples, establishing the communicative connection between the medical accessory and the host device may additionally comprise determining a quality of the communicative connection, as noted at 404. As will be further described below with respect to FIG. 11, determining the quality may comprise comparing a determined field strength to an expected field strength, wherein a weaker than expected field strength may indicate a misalignment, such as a misorientation or poor mechanical coupling of the medical accessory to the host device.

At 406, method 400 includes receiving accessory specific data from the medical accessory. The data storage device of the medical accessory may store accessory specific data thereof and once the communicative connection is established between the medical accessory and the host device, the accessory specific data may be transmitted to the host device via its reader device. The accessory specific data may include physical properties, such as length, diameter, material, and the like, operational properties, such as a commissioning date, and a remaining useful lifetime (or expected lifetime), and functional properties, such as accessory type, accessory model, bacterial and viral filtration efficiency (BFE/VFE), flow and vacuum data, and the like of the corresponding medical accessory. In some examples, the accessory specific data may also include accessory history data, including for example previous treatment operations, such as suction operations and nebulization operations, change in functionality/performance, number of previous hosts, and number of occlusion events.

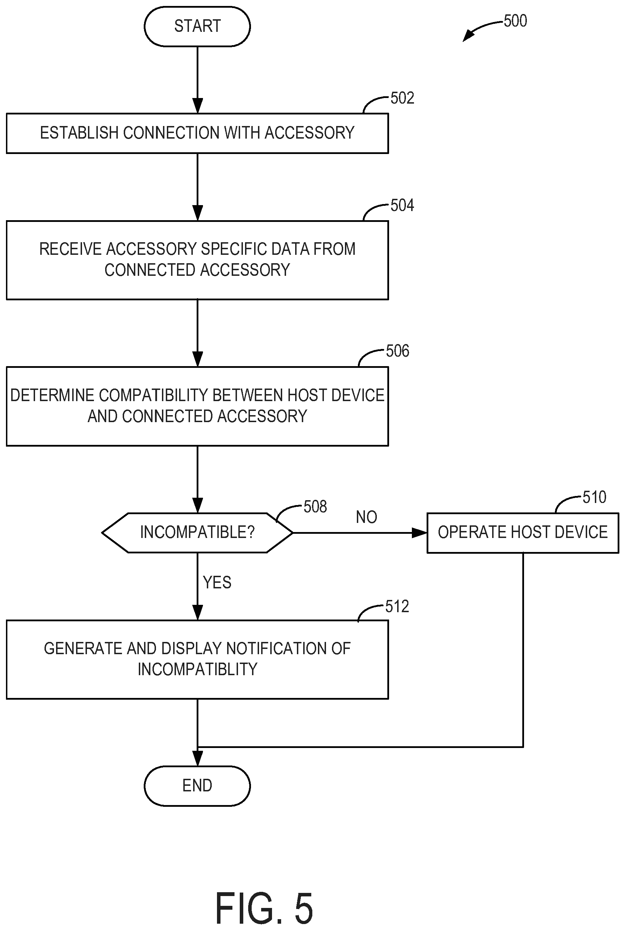

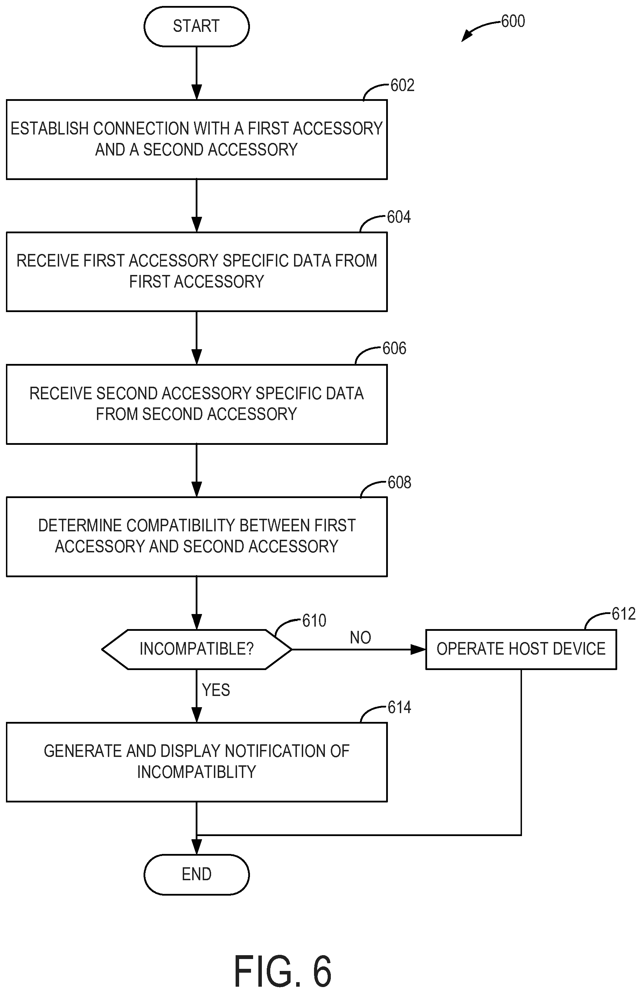

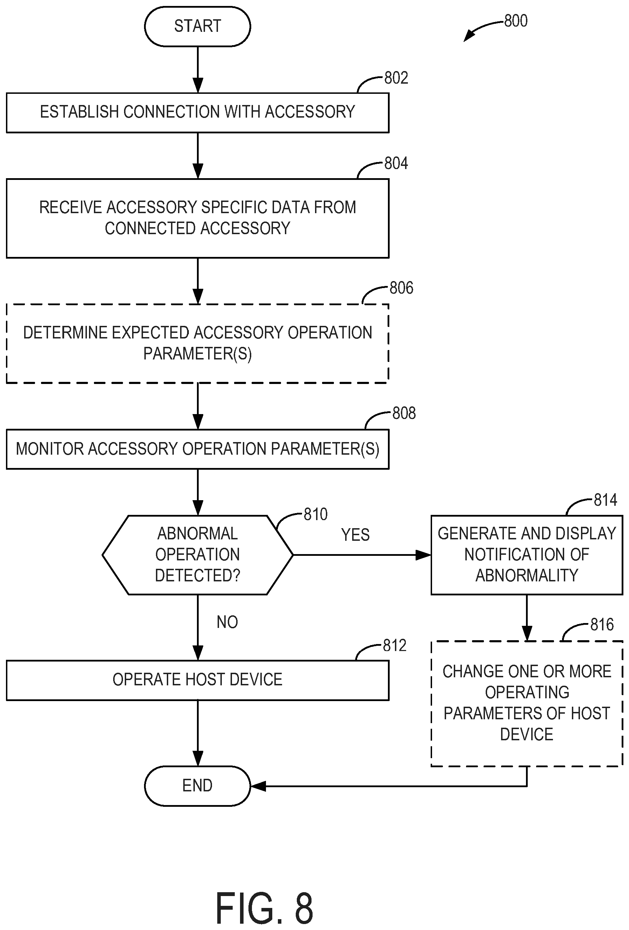

At 408, method 400 includes determining a compatibility and operational status of the medical accessory based on the accessory specific data. Determining compatibility of the medical accessory based on the accessory specific data may comprise evaluating a compatibility between the medical accessory and the host device, as will be described with respect to FIG. 5. Determining compatibility of the medical accessory may further include, in some examples, evaluating a compatibility between the medical accessory and another accessory connected to the host device, as will be described with respect to FIG. 6. Additionally, determining the operational status of the medical accessory may include evaluating one or more operational parameters to assess for possible malfunction of the medical accessory, as will be described with respect to FIG. 8.

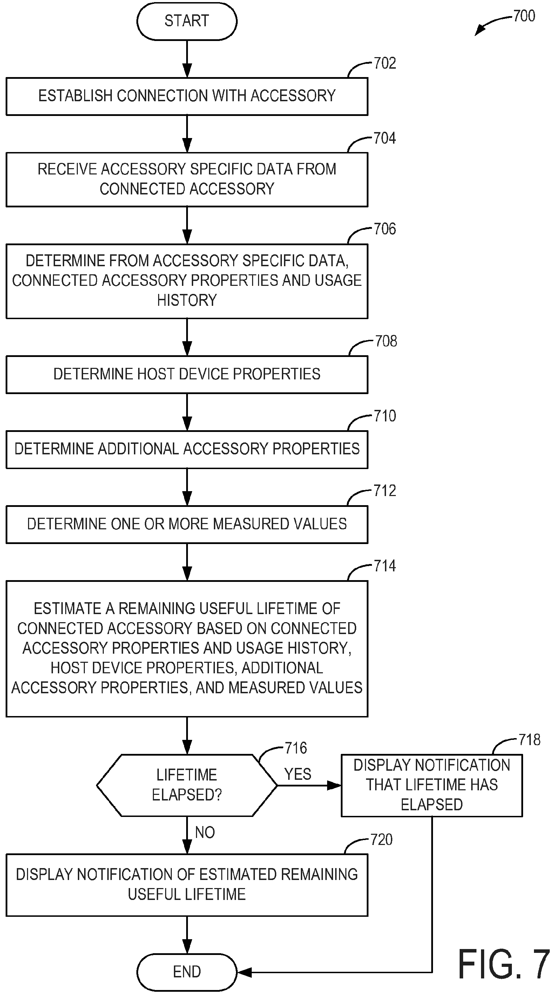

At 410, method 400 includes determining an accessory remaining useful lifetime (RUL) based on the accessory specific data. As will be further described with respect to FIG. 7, determining the remaining useful lifetime may include ingesting the accessory specific data, which may include previous treatment operations, a commissioning date, an expected lifetime, number of previous hosts, and the like, and determining a remaining useful lifetime. The remaining useful lifetime may be an estimate of a remaining duration for which the medical accessory may operate as expected and/or operate with an acceptable performance level.

In some examples, the remaining useful lifetime may be determined in response to the medical accessory being deemed compatible with the host device. As will be described below, if the medical accessory is deemed incompatible with the host device, the host device may output a notification of incompatibility.

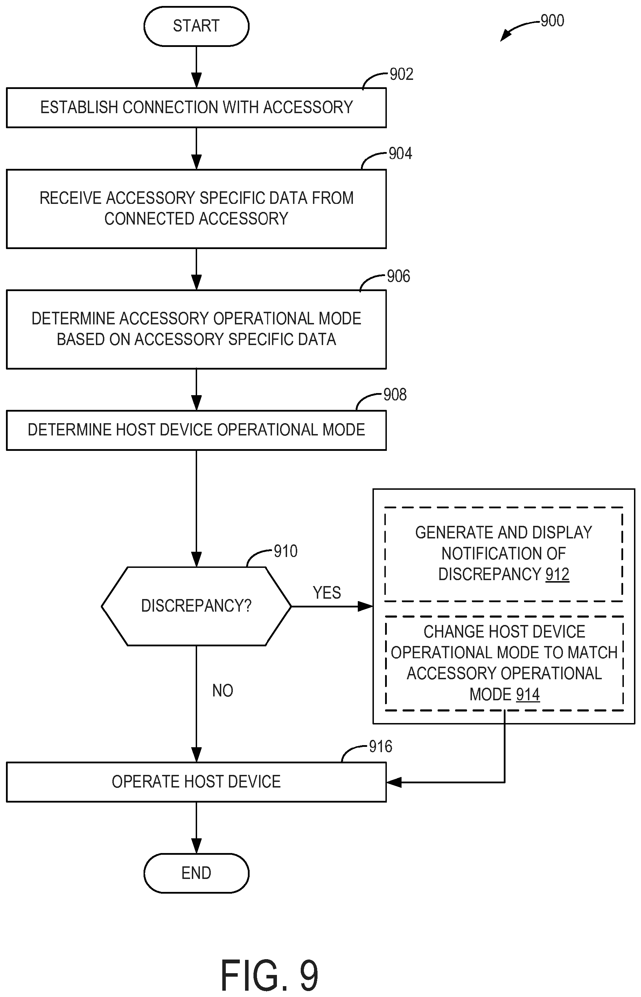

At 412, method 400 includes determining one or more host device operating parameters based on the accessory specific data. As will be further described with respect to FIG. 9, the one or more host device operating parameters may include an operating mode, such as adult mode, pediatric mode, or neonatal mode, sampling rate, tidal volume, inspiratory to expiratory ratio, and the like.

In some examples, the one or more host device operating parameters (e.g., the host device operational mode) may be determined in response to determination that the connected medical accessory is compatible and/or that the connected medical accessory has a remaining useful lifetime that has not yet elapsed. As will be described below, if the remaining useful lifetime is determined to have elapsed, the host device may output a notification that the lifetime is elapsed.

At 414, method 400 includes operating the host device according to the accessory specific data. In particular, the operating modes as determined at 410 may be employed during operation of the host device. In addition, the host device may provide notifications to the user via a user interface based on the accessory specific data. For example, a remaining useful lifetime determined based on the accessory specific data may be displayed to the user via a user interface. Further, a notification that the remaining useful lifetime is determined to be elapsed (e.g., no remaining useful lifetime) may be displayed to the user via the user interface. Further still, determined operating mode contradictions or determinations of incompatibility may be displayed to the user via notifications. As an example, a user may set the host device operating mode as adult mode and the host device may detect the medical accessory as a neonatal accessory. In some examples, the operating mode may be automatically changed to neonatal to match the connected neonatal accessory. In other examples, a notification of a mismatch may be presented via the user interface, which may prompt the user to either manually change the host device operating mode or to exchange the medical accessory, depending on the circumstance.

Turning now to FIG. 5, a flowchart illustrating a method 500 for determining compatibility between a host device and an accessory is shown. The method 500 herein described may be executed by a host device. In particular, the method 500 may be executed by one or more processors according to instructions stored in memory of the host device, e.g., by processor(s) 104 according to instructions stored in memory 106). The method 500 is herein described with respect to the system components as described with respect to FIGS. 1-3, though it should be understood that the method 500 may apply to other systems as well. The host device as referenced with respect to method 500 may be a gas analyzer and the medical accessory may be one of a water trap and a sampling line. It should also be understood that the method 500 may be performed for each accessory coupled to the host device, in some examples.

At 502, method 500 includes establishing a communicative connection with an accessory. As described above, the host device may establish the communicative connection between the host device and the medical accessory when the medical accessory is oriented such that its memory storage device (e.g., NFC tag) is positioned within range of a reader device of the host device (e.g., within a field of the reader device).

At 504, method 500 includes receiving accessory specific data of the connected medical accessory from the connected medical accessory. As described herein, the medical accessory may include a memory device, such as an NFC tag, that stores data relating to the medical accessory. The stored data may include data related to the medical accessory properties as well as expected host devices and host device properties that the medical accessory may be compatible with and/or incompatible with.

At 506, method 500 includes determining compatibility between the host device and the connected medical accessory. The host device may have known host device properties, such as model, type, available operational modes, demanded flow rates, gases used for analysis, gas humidity used, and the like. The available operational modes may include adult mode, pediatric mode, and neonatal mode. The host device properties may be compared to the accessory specific data to determine whether the connected medical accessory is compatible with the host device. If one or more of the host device properties are indicated as incompatible with the medical accessory, host device may be determined to be incompatible with the medical accessory. As an example, the host device may be a gas analyzer specifically configured for use in volatile anesthetic monitoring, which demand specific water traps and/or sampling lines that do not interfere with anesthetic agent detection. In examples in which a connected water trap is not one of these specialized water traps, the connected water trap may be determined to be incompatible with the host device.

As another example, some gas analyzers can demand a specific sample flow rate or range of sample flow rates. A connected sampling line may store data of its available or expected sample flow rates and expected differential pressure in the memory device, which is transmitted to the host device once connected. If the available sample flow rates include the specific sample flow rate demanded by the gas analyzer, the connected sampling line may be considered compatible with the host device.

In practice, a plurality of parameters may be checked in order to determine compatibility between the host device and the connected medical accessory. In some examples, if one parameter is determined to be incompatible, the medical accessory may be determined to be incompatible. The parameters considered may include model and make of the medical accessory and the host device, such as may be derived from a product identifier (e.g., a UID, encrypted authentication key, etc.), available sample flow rates vs demanded sample flow rates, water trap available volume, type of water trap membrane (e.g., which may affect moisture and BDE/VFE filtering efficacy), accessory material, gases used by sampling line, operational mode, accumulated number of specific events at the host device side, such as zeroing, and more.

At 508, method 500 includes judging if the host device and the connected medical accessory are incompatible. As described above, if one of the compared parameters are incompatible, the host device may be considered to be incompatible with the connected medical accessory. If all parameters are compatible, the host device may be considered to be compatible with the connected medical accessory. If the host device and the connected medical accessory are compatible (e.g., not incompatible) (NO at 508), method 500 proceeds to 510. If the host device and the connected medical accessory are incompatible (YES at 508), method 500 proceeds to 512.

At 510, method 500 includes operating the host device. As an example, the host device may be a gas analyzer and may be operated to analyze parameters like flow, pressure, volume, and gas concentration to ensure accurate ventilation delivery by a ventilator system. The host device may be operated according to operational parameters determined based on the accessory specific data, as will be further discussed below.

At 512, method 500 includes generating and displaying a notification of incompatibility. For example, the host device may comprise a user interface that may display visual notifications via a graphical user interface. Other or additional notifications, such as audible notifications (e.g., beeps, speech, etc.) may also be used. The notification may indicate to the user that the connected medical accessory is incompatible with the host device. In some examples, the notification may indicate a reason for incompatibility. Further, in some examples, the notification may indicate what the medical accessory is (e.g., water trap vs sampling line) to indicate to the user which accessory is incompatible if more than one accessory is connected at a time.

Determining compatibility for each connected accessory in this manner may reduce unreliable gas readings resultant from issues such as leakage with ill-fitting couplings, disconnections under pressure changes from ill-fitting couplings, changes in flow rate that may affect gas readings, longer response times (e.g., when too long of a sampling line is connected), altered gas concentrations resultant from non-optimal sampling line length, non-ideal sample flow resultant from non-optimal sampling line diameter, unreliable or incorrect gas concentration readings resultant from gas absorption, for example when a connected sampling line is formed of a material that absorbs certain gases to be sampled, and more. Humans are prone to error in selection of accessories to be connected, which may ultimately result in the above issues if an incompatible accessory is used during operation of the gas analyzer. Compatibility determination as outlined herein may mitigate this issue, leading to more accurate readings and longer useful lifetimes of the accessories.

Turning now to FIG. 6, a flowchart illustrating a method 600 for determining compatibility between two accessories is shown. The method 600 herein described may be executed by a host device to which the two accessories are coupled. In particular, the method 600 may be executed by one or more processors according to instructions stored in memory of the host device, e.g., by processor(s) 104 according to instructions stored in memory 106). The method 600 is herein described with respect to the system components as described with respect to FIGS. 1-3, though it should be understood that the method 600 may apply to other systems as well. The host device as referenced with respect to method 600 may be a gas analyzer and the accessories may be a water trap and a sampling line, in one non-limiting example.

At 602, method 600 includes establishing a communicative connection with a first accessory and a second accessory. As described above, the host device may establish the communicative connection between the host device and the medical accessory when the respective accessory is oriented such that its memory storage device (e.g., NFC tag) is positioned within range of a reader device of the host device (e.g., within a field of the reader device). As a non-limiting example, the first accessory may be a water trap and the second accessory may be a sampling line.

At 604, method 600 includes receiving first accessory specific data from the first accessory. As described herein, the first accessory may include a memory device, such as an NFC tag, that stores data relating to the medical accessory. The stored data may include data related to the first accessory properties as well as other accessory demands. For example, a water trap may comprise a particular type of filter (e.g., a hydrophobic filter) that may interact with certain sampling line materials and thus the properties of the water trap stored in its memory device may indicate the sampling line materials that are incompatible with the water trap's filter.

At 606, method 600 includes receiving second accessory specific data from the second accessory. As described herein, the first accessory may include a memory device, such as an NFC tag, that stores data relating to the medical accessory. The stored data may include data related to the second accessory properties as well as other accessory demands. For example, a sampling line may be configured for either a dry environment or humid environment and thus this property of the sampling line may inform compatible properties of water traps, for example whether the sampling line is optimized for dry or humid environments.

At 608, method 600 includes determining compatibility between the first accessory and the second accessory. The first and second accessory properties received from the first and second accessories may be compared in order to determine compatibility between the two. Properties considered for compatibility by include, but are not limited to, accessory materials, length/diameter of the sampling line, ventilation modes (e.g., including adult vs pediatric vs neonatal), target environment (e.g., dry vs humid), gas capabilities (e.g., which gases the medical accessory is capable of handling), and the like. The properties of the first accessory may be compared to the properties of the second accessory to determine whether the connected accessories are compatible with each other.

In practice, a plurality of parameters may be checked in order to determine compatibility between the host device and each connected accessory as well as between the connected accessories. In some examples, if one parameter is determined to be incompatible with either the host device or the other connected accessory, the medical accessory may be determined to be incompatible.

At 610, method 600 includes judging if the first and second accessories are incompatible. As described above, if one of the compared parameters are incompatible, the first accessory may be considered to be incompatible with the second accessory. If all parameters are compatible, the first accessory may be considered to be compatible with the second accessory. If accessories are compatible (e.g., not incompatible) (NO at 610), method 600 proceeds to 612. If the accessories are incompatible (YES at 610), method 600 proceeds to 614.

At 612, method 600 includes operating the host device. As an example, the host device may be a gas analyzer and may be operated to analyze parameters like flow, pressure, volume, and gas concentration to ensure accurate ventilation delivery by a ventilator system. The host device may be operated according to operational parameters determined based on the first and second accessory specific data, as will be further discussed below.

At 614, method 600 includes generating and displaying a notification of incompatibility. For example, the host device may comprise a user interface that may display visual notifications via a graphical user interface. Other or additional notifications, such as audible notifications (e.g., beeps, speech, etc.) may additionally or alternatively be used. The notification may indicate to the user that the first and second accessories are incompatible. In some examples, the notification may indicate a reason for incompatibility. Further, in some examples, incompatibility between accessories and incompatibility, if present, between accessory and host device may be considered. For example, if the first accessory is incompatible with the second accessory and the first accessory is also incompatible with the host device (while the second accessory is compatible with the host device), the notification may indicate that the first accessory is the incompatible device. In this way, the user may only have to remove and replace one accessory rather than removing both accessories to determine which one to exchange with a different accessory of the same accessory type.

Similar to determining compatibility between the host device and an accessory, determining accessory-accessory compatibility may mitigate human errors in accessory selection, thereby reducing errors, inconsistencies, or inaccuracies in gas sampling and readings and increasing the longevity of lifetime of the accessories.

Turning now to FIG. 7, a flowchart illustrating a method 700 for determining a remaining useful lifetime of a connected accessory is shown. The method 700 herein described may be executed by a host device to which the medical accessory is connected. In particular, the method 700 may be executed by one or more processors according to instructions stored in memory of the host device, e.g., by processor(s) 104 according to instructions stored in memory 106). The method 700 is herein described with respect to the system components as described with respect to FIGS. 1-3, though it should be understood that the method 700 may apply to other systems as well. The host device as referenced with respect to method 700 may be a gas analyzer and the connected medical accessory may be a water trap or a sampling line, in one non-limiting example.

At 702, method 700 includes establishing a communicative connection with an accessory coupled to the host device. As described above, the communicative connection between the host device and the medical accessory may be established when the medical accessory is oriented such that its memory storage device (e.g., NFC tag) is positioned within range of a reader device of the host device (e.g., within a field of the reader device).

At 704, method 700 includes receiving accessory specific data from the connected medical accessory. As described herein, the medical accessory may include a memory device, such as an NFC tag, that stores data relating to the medical accessory. The stored data may include data related to the medical accessory properties.

At 706, method 700 includes determining, from the accessory specific data, connected accessory properties and usage history. The connected medical accessory properties may comprise physical properties of the medical accessory, operational properties of the medical accessory, and/or functional properties of the medical accessory. As examples, physical properties, for example of a sampling line, may comprise length, diameter, and material the medical accessory is made of, operational properties may comprise commissioning date and expected lifetime of the medical accessory, and functional properties may comprise type, model, flow and vacuum, and/or bacterial and viral filtration efficiency (BFE/VFE) (e.g., for water traps). The usage history properties may comprise previous treatment operations (e.g., numbers, durations, etc. of treatment operations), such as suction operations and nebulization operations, change in functionality/performance, number of occlusion events, and number of previous hosts. Additionally, the usage history may include angular data from an accelerometer included in the medical accessory, the angular data indicating orientations that the medical accessory has had over time, which may affect how particulates and liquids have built up.

At 708, method 700 includes determining host device properties. The host device may know (e.g., have stored in memory) properties thereof. These host device properties may comprise physical properties, functional properties, and an operational mode. The operational mode may be a currently selected operational mode of one or more available operational modes. The selected operational mode may be selected via user input, based on the accessory specific data, or both. The physical properties of the host device may include number and type of physical/fluid connections available and host device components. The functional properties may comprise type and model.

At 710, method 700 includes determining additional accessory properties. As described herein, in some examples more than one accessory may be connected to the host device, such as both a water trap and a sampling line. The additional accessory properties may be properties of another connected accessory and may include accessory type, physical properties, material, and the like.

As a non-limiting example, the connected medical accessory may be a water trap and an additional accessory may be a sampling line. The sampling line properties may include length, diameter, material, commissioning date, expected lifetime, model, type, sampling flow, gas capabilities, accessory humidity rating, flow resistance, and intended flow range. The water trap properties may comprise type, BFE/VFE, and gas capabilities.

At 712, method 700 includes determining one or more measured values. The one or more measured values may be determined during use of the system. As described with respect to FIG. 1, the host device may comprise a measurement system including one or more sensors, such as a flow sensor and a differential pressure sensor, configured to measure values such as flow rate, differential pressure, and resistance. These measured values may indicate how well the medical accessory is operating. For example, flow resistance may increase as a result of accumulation of water and particulates within a sampling line or a filter of a water trap, and increased flow resistance may reduce the efficacy of said sampling line and/or water trap.

At 714, method 700 includes estimating a remaining useful lifetime of the connected medical accessory based on the connected medical accessory properties and usage history, the host device properties, the additional accessory properties, and the measured values. In one example, estimating the remaining useful lifetime may comprise inputting the connected medical accessory properties and usage history, the host device properties, the additional accessory properties, and the measured values into a machine learning model that is trained to ingest such properties and output an estimated remaining useful lifetime. A trained machine learning model may be an adaptive model capable of determining a remaining useful lifetime in a variety of scenarios. In another example, estimating the remaining useful lifetime may comprise employing a mathematical algorithm, a plurality of mathematical algorithms, or the like. For example, a degradation curve algorithm may be used to estimate the remaining useful lifetime.

In examples when a mathematical algorithm is used to determine the remaining useful lifetime, present measurement data may be combined with initial values, previous events, and moving average in order to generate an estimate of the remaining useful lifetime.

At 716, method 700 includes judging if the lifetime of the connected medical accessory has elapsed. As an example, the estimated remaining useful lifetime may be zero or otherwise an indicator that there is no lifetime left for the medical accessory. If the lifetime has elapsed, method 700 proceeds to 718. If the lifetime has not elapsed, method 700 proceeds to 720. In some examples, determination of whether the lifetime has elapsed may consider a useful margin, whereby an estimated remaining useful lifetime below a threshold is considered an elapsed lifetime. For example, in the context of surgical operations, a remaining useful lifetime of less than an hour may be deemed elapsed because most surgical operations have a duration of longer than an hour.

At 718, method 700 includes displaying a notification that the lifetime of the connected medical accessory has elapsed. As described herein, the host device may comprise a user interface by which notifications may be displayed. The notification may be a textual notification, an audible notification (e.g., a beep), or other visual notification (e.g., a light). The notification may inform the user that the lifetime of the connected medical accessory has elapsed and thus that the connected medical accessory is recommended to be replaced.

At 720, method 700 includes displaying a notification of the estimated remaining useful lifetime. The notification may be a textual notification, an audible notification (e.g., a beep), or other visual notification (e.g., a light) displayed by the host device, for example via the user interface. The notification may inform the user of the lifetime of the connected medical accessory, thus allowing the user to plan for when to replace the connected medical accessory.

Overall, estimation of the remaining useful lifetime of a connected accessory may reduce the instance of usage of expired accessories. For example, manual tracking of remaining useful lifetime based on the expected lifetime provided by a manufacturer is prone to user errors. Further, expected lifetimes provided by the manufacturer may not account for the actual usage scenario of the medical accessory and thus, even if the expected lifetime is adhered to by users, the medical accessory may have reduced performance because of its actual usage scenario. Estimating the remaining useful lifetime as herein described may account for the actual usage scenario, including the properties of the medical accessory itself, the host device to which the medical accessory is connected, and other accessories also in use/connected to the host device.

In some examples, remaining useful lifetime may only be determined for accessories deemed compatible with the host device. For example, compatibility with the gas analyzer for a connected accessory may be determined first and then, if compatible, the remaining useful lifetime of that accessory may be determined.

FIG. 8 shows a flowchart illustrating a method 800 for determining operational status of a connected accessory. The method 800 herein described may be executed by a host device to which the medical accessory is connected. In particular, the method 800 may be executed by one or more processors according to instructions stored in memory of the host device, e.g., by processor(s) 104 according to instructions stored in memory 106). The method 800 is herein described with respect to the system components as described with respect to FIGS. 1-3, though it should be understood that the method 800 may apply to other systems as well. The host device as referenced with respect to method 800 may be a gas analyzer and the connected medical accessory may be a water trap or a sampling line, in one non-limiting example.

At 802, method 800 includes establishing a communicative connection with an accessory coupled to the host device. As described above, the communicative connection between the host device and the medical accessory may be established when the medical accessory is oriented such that its memory storage device (e.g., NFC tag) is positioned within range of a reader device of the host device (e.g., within a field of the reader device).

At 804, method 800 includes receiving accessory specific data from the connected medical accessory. As described herein, the medical accessory may include a memory device, such as an NFC tag, that stores data relating to the medical accessory. The stored data may include data related to the medical accessory properties and parameters, such as physical properties, functional properties, operational properties, and/or usage history, as herein described.

At 806, method 800 optionally includes determining expected accessory operation parameters. As an example, flow resistance may be a monitored for one or both of a sampling line and a water trap. For example, flow resistance for a sampling line may depend on length and diameter of the line and may be affected by particulate buildup or other factors over time. Similarly, flow resistance for a water trap may depend on the filter therein and may be affected by water accumulation, patient excretions like saliva and phlegm, residuals accumulation, and filter clogging over time. Expected patterns of flow resistance may be stored in the accessory specific data, for example as an expected average flow resistance. This parameter may be transmitted to the host device when the medical accessory is connected to the host device. The host device may be configured to monitor the flow resistance through the system, for each of the sampling line and the water trap individual or for both at the same time.

In some examples however, expected accessory operation parameters may not be available or included in the accessory specific data. In such examples, the host device may assess accessory operation status without the expected accessory operation parameters, as will be described below.

At 808, method 800 includes monitoring accessory operation parameter(s). For example, the host device may comprise one or more sensors positioned within the device or along the sampling line to monitor operational parameters. In some examples, the monitored parameters, such as flow resistance, humidity and water content, pressure levels, and temperature, may be monitored with respect to the expected accessory operation parameters. In other examples, the monitored parameters may be considered on their own.

At 810, method 800 includes judging whether an abnormal operation state is detected. Using flow resistance as a use-case example, in one example, a measured average and/or moving average flow resistance may be analyzed to detect rapid changes.

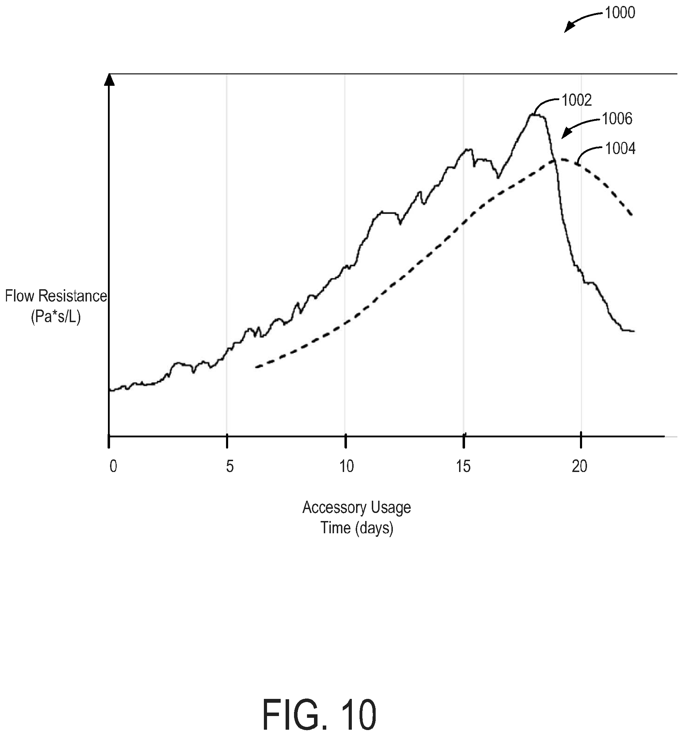

Turning briefly to FIG. 10, a graph 1000 of measured flow resistance over time is shown. Flow resistance, shown in graph 1000 in centimeters of water per liter per second though other units such as Pascal seconds per liter or millimeters of Mercury per liter per second may also be used, is shown in a y-axis. Accessory usage time, in days, is shown on an x-axis.

The graph 1000 comprises a measured flow resistance plot 1002 and an average flow resistance plot 1004. The average flow resistance plot 1004 may be a moving average that accounts for noise or other interferences in the unfiltered data plotted on the measured flow resistance plot 1002. A rapid decrease in flow resistance 1006 is present around 16 days of usage. The rapid decrease in flow resistance 1006 may reflect some event that rapidly affected the flow resistance, such as a puncture in the water trap membrane, accessory orientation change, and increased amount of sensor zeroing inside the host module. This rapid decrease in flow resistance 1006 may be considered an abnormal operation state (e.g., a malfunction or a contamination) and may be detected by the host device.

In another example, a rapid increase in flow resistance may be detected, which may indicate an event such as a nebulization.

Returning to FIG. 8, detection of an abnormal operation state may suggest that some malfunction or contamination has occurred that may affect performance of the medical accessory and/or the host device. For example, water trap integrity affects the operation of the gas analyzer. If the water trap membrane is punctured, water and/or liquids and bacteria may eventually enter the host device and render it inoperative as well as potentially non-repairable. Detection of such events through monitoring of water trap operation parameters like flow resistance may allow the host device to more readily detect abnormal operations.

In some examples, when expected operation parameters are provided by the medical accessory, the host device may compare measured parameters, such as measured or averaged flow resistances, to the expected operation parameters. This may allow the host device to detect if the medical accessory is underperforming, e.g., has a statistically significantly higher average flow resistance compared to its expected operation, which may indicate a malfunction, such as a blockage or leakage. As an example, if a new (e.g., new out of package) water trap is coupled to the host device, but the host device is still observing increased flow resistance similar to the recently decoupled water trap (e.g., flow resistance expected at end of useful lifetime), the host device may determine that a malfunction is occurring in the new water trap or in the host device.

If abnormal operation is detected, method 800 proceeds to 814. If no abnormal operation is detected, method 800 proceeds to 812.

At 812, method 800 includes operating the host device. For example, the host device may continue to operate as is without changes.

At 814, method 800 includes generating and displaying a notification of the abnormality. For example, the host device may include a user interface configured to display textual, audio, or visual notifications to users. As an example, a notification indicating that an abnormal operation was detected may be displayed to a user of the host device. In some examples, the notification may indicate what the abnormality is. For example, the user may have intentionally altered an orientation of the sampling line, which may temporarily or permanently affect operational parameters of the sampling line and/or water trap. The user may thus know to ignore a detected abnormality that is generated following this intentional alteration. In another example, the user may use the notification to determine what accessory to check for malfunction based on the notification.

In some examples, detection of an abnormal state may include determination of different abnormal states. For example, a detected combination of slightly or mildly reduced flow rate, diluted gas concentrations, and reduced pressure differential may indicate that an air leak in the sampling line or water trap has been introduced (e.g., via a puncture, wall defect, etc.). In comparison, a significantly decreased flow rate, an increased pressure differential, and a rapidly rising flow resistance may indicate that the sampling line is clogged or otherwise obstructed. In this way, not only can an abnormal state of a medical device accessory be detected, the type of abnormal state may also be determined.

At 816, method 800 optionally includes changing one or more operating parameters of the host device in response to detection of the abnormal state of the medical accessory (e.g., a malfunction or contamination of the medical accessory). As a non-limiting example, a gas flow rate may be reduced or a valve on a gas line of the host device (e.g., the gas analyzer) may be closed. For example, when a contamination is detected based on the observed operation of one or more medical accessories, the gas flow rate may be reduced and/or the valve may be closed to reduce the possibility of drawing debris or liquids into internal components of the gas analyzer and of overloading internal filters that could degrade the sampling pump.

Thus, operational state of connected accessories may be monitored in real-time, detecting abnormal states more quickly than is possible for a human to do by observation. Further, detection of the type of abnormal state and intervention therefor, such as closing of the valve or reducing the flow rate, may be executed quicker than a human could do so once they observe an abnormality. In this way, issues with accessories may be addressed more quickly, reducing potential issues or degradation to the system that would arise from an improperly functioning system.

Turning now to FIG. 9, a flowchart illustrating a method 900 for determining operational mode of a host device based on a connected accessory is shown. The method 900 herein described may be executed by a host device to which the medical accessory is connected. In particular, the method 900 may be executed by one or more processors according to instructions stored in memory of the host device, e.g., by processor(s) 104 according to instructions stored in memory 106). The method 900 is herein described with respect to the system components as described with respect to FIGS. 1-3, though it should be understood that the method 900 may apply to other systems as well. The host device as referenced with respect to method 900 may be a gas analyzer and the connected medical accessory may be a water trap or a sampling line, in one non-limiting example.

At 902, method 900 includes establishing a communicative connection with an accessory coupled to the host device. As described above, the communicative connection between the host device and the medical accessory may be established when the medical accessory is oriented such that its memory storage device (e.g., NFC tag) is positioned within range of a reader device of the host device (e.g., within a field of the reader device).

At 904, method 900 includes receiving accessory specific data from the connected medical accessory. As described herein, the medical accessory may include a memory device, such as an NFC tag, that stores data relating to the medical accessory. The stored data may include data related to the medical accessory properties and parameters, such as physical properties, functional properties, operational properties, and/or usage history, as herein described.

At 906, method 900 includes determining an accessory operational mode based on the accessory specific data. As described herein, the accessory specific data may include data of a model, a type, and the like. The model and type may indicate the mode for which the medical accessory is intended to be used. For example, the medical accessory may be a neonatal water trap, the operational mode being neonatal.

At 908, method 900 includes determining a host device operational mode. The host device may be configured to operate in one of one or more available operational modes. In some examples, one of the one or more available operational modes may be selected and this may be the current host device operational mode. The operational mode may be selected as a default mode or from user input, in some examples.

At 910, method 900 includes determining if there is a discrepancy between the host device operational mode and the medical accessory operational mode. There may be a discrepancy when the operational modes are different. As an example, the medical accessory operational mode may be neonatal, determined based on the model and type of the medical accessory obtained from the accessory specific data. When the host device operational mode is also neonatal, there may not be a discrepancy. When the host device operational mode is one of adult and pediatric, there may be a discrepancy. When there is a discrepancy (YES at 910), method 900 proceeds to one of 912 and 914. When there is not a discrepancy (NO at 910), method 900 proceeds to 916.

In response to a discrepancy in operational mode being detected, method 900 may include: 1) generating and displaying a notification of the discrepancy, as noted at 912; or 2) changing the host device operational mode to match the medical accessory operational mode. Changing the host device operational mode may include updating the currently selected operational mode to the mode of the medical accessory. The operational mode may influence operational parameters such as flow rate, sampling rate, trigger sensitivity, pressure and flow calibration, measurement adjustments, alarm thresholds, and the like. For example, a neonatal mode may include a lower sampling rate compared to an adult mode (e.g., 50-150 mL/min compared to 200-500 mL/min).

Similar to as described herein, the notification may be displayed via a user interface of the host device and may indicate that the connected medical accessory is a model and type intended to be used with an operational mode of the host device that does not match the current operational mode of the host device. In some examples, the notification may include information of the mismatch, allowing the user to make a choice of either changing the host device operational mode or exchanging the medical accessory (e.g., in the even the medical accessory of a particular operational mode was erroneously connected to the host device). In some examples, the notification may include a selectable prompt to the user via which the user may indicate that the host device operational mode is to be changed to match the medical accessory.

At 916, method 900 includes operating the host device according to the selected host device operational mode. Method 900 may include operating the host device when no discrepancy is detected at 910, and/or following changing of the operational mode if a discrepancy is detected at 910. For example, following 914 where the mode is changed to match the medical accessory, method 900 may include operating the host device according to the newly selected operational mode.

In this way, operational mode of the host device may be determined specifically for the connected accessories. Further, mismatches between user-selected operative mode and model of an accessory may be quickly determined, either to update the selected mode of the host device to match the medical accessory and/or to notify the user that a mismatch is detected, thereby reducing the potential for operating the host device in a mode that is incompatible with the connected medical accessory. This may overall reduce inaccurate readings and operational issues that would arise from operating the host device in a manner incompatible with the connected medical accessory, which in turn may increase the lifetime of the medical accessory.

Turning now to FIG. 11, a flowchart illustrating a method 1100 for determining quality of a communicative connection between a medical accessory and a host device is shown. The method 1100 herein described may be executed by the host device to which the medical accessory is coupled. In particular, the method 1100 may be executed by one or more processors according to instructions stored in memory of the host device, e.g., by processor(s) 104 according to instructions stored in memory 106). The method 1100 is herein described with respect to the system components as described with respect to FIGS. 1-3, though it should be understood that the method 1100 may apply to other systems as well. The host device as referenced with respect to method 1100 may be a gas analyzer and the connected medical accessory may be a water trap or a sampling line, in one non-limiting example.

At 1102, method 1100 includes establishing a communicative connection with the medical accessory. As described above, the communicative connection between the host device and the medical accessory may be established when the medical accessory is oriented such that its memory storage device (e.g., NFC tag) is positioned within range of a reader device of the host device (e.g., within a field of the reader device).

At 1104, method 1100 optionally includes determining an expected field strength. In some examples, a reading system of the host device may have a known expected field strength for its reader. The expected field strength may be based on an ideal placement (e.g., positioning and orientation) of the medical accessory with respect to the host device. For example, a typical field strength of an NFC reader at close range (e.g., 0-4cm) may be between 3 and 7.5 A/m, wherein a higher A/m reading indicates closer positioning of the NFC tag (e.g., in this case the memory device of the medical accessory) to the reader (e.g., the reader of the host device). Thus, the host device may have a known expected field strength for the communicative connection between the memory device of the medical accessory and the host device based on an ideal position and orientation of the medical accessory with respect to the host device, for example based on an expected distance between the memory storage device and the reader of the host device.

At 1106, method 1100 includes determining a field strength of the communicative connection between the medical accessory and the host device. In some examples, field strength may be inferred by observing how well the tag communicates with the reader. In other examples, a near-field probe may be included in the reading system to measure the magnetic field strength.

In some examples, the determined field strength may be compared directly to a predefined threshold value. For example, a standard minimum field strength of 1.5 A/m may be set for the host device. The threshold value may be configurable to allow system developers and/or manufacturers to define a desired minimum field strength.

In other examples, the determined field strength may be compared to the expected field strength to determine a difference therebetween, as noted at 1108. A threshold difference value may be predefined in such a case, wherein if the difference between the determined field strength and the expected field strength is greater than the threshold, the medical accessory may be said to be malpositioned or malaligned.

At 1110, method 1100 includes determining if the determined field strength is less than the threshold value (or if the determined difference between the expected field strength and determined field strength is greater than the threshold difference). A determined field strength less than the threshold value or a determined difference between the expected field strength and the determined field strength is great than the threshold difference, the connection quality may be weaker than desired, which may indicate that the medical accessory is malpositioned, misaligned, mal oriented, or otherwise not connecting well enough to the reader of the host device. When the connection quality is not weak (NO at 1110), method 1100 proceeds to 1114 to execute one or more determinations, such as of compatibility, operational status, remaining useful lifetime, and the like as herein described.

When the connection quality is weak (YES at 1110), method 1100 proceeds to 1112, where method 1100 includes generating and displaying a notification of poor connection quality for the medical accessory. Similar to as described herein, the notification may be displayed via a user interface of the host device and may indicate that the determined field strength between the medical accessory and the host device is lower than desired or expected. In some examples, suggestions for reasons for the poor connection quality, such as malpositioning or misalignment of the accessory may also be included in the notification to guide the user.

The methods and systems described herein include a system wherein accessories for a gas analyzer system comprise memory storage device, such as NFC tags, and the gas analyzer comprises a reading system, wherein a communicative connection between an accessory and the gas analyzer (e.g., host device) is established when the NFC tag of the medical accessory is within range of the reading system of the host device. Data from the medical accessory stored in the NFC tag may be transmitted to the gas analyzer once the communicative connection is established. The technical effect of the systems and methods disclosed herein is that useful usage time for accessories may be prolonged by ensuring accessory-host and accessory-accessory compatibility. Further, ensuring compatibility with the host device may reduce degradation to the host device itself, thereby minimizing down time and repair and maintenance needs. Further, ensuring compatibility with the host device and other accessories may reduce inaccurate readings, leakages, and more that may otherwise affect performance of the system.

Further still, the methods and systems disclosed may more accurately calculate remaining useful lifetime of connected accessories by considering the medical accessory's properties and usage history, the host device's properties, and properties of other connected accessories. In this way, the lifetimes of the accessories may be more accurately monitored, reducing potential for usage of an expired accessory which may otherwise affect performance of the system as a whole.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. A gas analyzer system (200), comprising:
a gas analyzer (102, 7) having a reading system (116, 40) and one or more processors (104) configured to execute instructions stored in memory (106); and
at least one medical accessory (120, 24, 27) comprising a data storage system (122), wherein the one or more processors are configured to:
establish a communicative connection with the data storage system of the at least one medical accessory (402);
in response to establishing the communicative connection, receive accessory specific data of the at least one medical accessory by the reading system from the data storage system (406);
determine compatibility of the at least one medical accessory and the gas analyzer based on the accessory specific data (408);
determine a remaining useful lifetime of the at least one medical accessory based on at least the accessory specific data (410);
in response to the at least one medical accessory and the gas analyzer being compatible, operate the gas analyzer based on the accessory specific data (508, 510); and
in response to the at least one medical accessory and the gas analyzer being incompatible, generate a first notification of incompatibility (508, 512).

2. The gas analyzer system of claim 1, wherein the one or more processors are further configured to generate a second notification of the remaining useful lifetime of the at least one medical accessory to a user interface of the gas analyzer (720).

3. The gas analyzer system of claim 1, wherein the one or more processors are configured to determine the remaining useful lifetime of one of the at least one medical accessory based on the accessory specific data of the one of the at least one medical accessory, properties of the gas analyzer, accessory specific data of other accessories of the at least one medical accessory, and one or more measured values (714), wherein the accessory specific data of the one of the at least one medical accessory comprises physical properties, functional properties, operational properties, and usage history of the one of the at least one medical accessory, and the one or more measured values comprise flow rate, differential pressure, and flow resistance.

4. The gas analyzer system of claim 1, wherein the accessory specific data of the at least one medical accessory includes a model and a type, wherein the model and the type correspond to an operational mode of the gas analyzer (704, 706).

5. The gas analyzer system of claim 4, wherein to operate the gas analyzer based on the accessory specific data when the at least one medical accessory is compatible with the gas analyzer, the one or more processors are configured to operate the gas analyzer according to the operational mode that corresponds to the model and type of the at least one medical accessory (510).

6. The gas analyzer system of claim 1, wherein the communicative connection between the at least one medical accessory and the gas analyzer is established when the data storage system of the at least one medical accessory is within a field of the reading system of the gas analyzer (402).

7. The gas analyzer system of claim 1, wherein the reading system (116, 40) and the data storage system (122) are near field communication (NFC) tags.

8. The gas analyzer system of claim 1, wherein, when the at least one medical accessory comprises a first accessory and a second accessory, the one or more processors are further configured to determine compatibility between the first accessory and the second accessory based on accessory specific data of the first accessory and accessory specific data of the second accessory.

9. The gas analyzer system of claim 1, wherein the at least one medical accessory comprises one or more of a water trap (27) and a sampling line (24).

10. A method for a gas analyzer system (200) comprising a gas analyzer (102, 7) configured to couple to one or more medical accessories (120, 24, 27), comprising:
establishing a communicative connection with one or more of the one or more medical accessories (402), each of the one or more of the one or more medical accessories comprising a memory device (122) storing accessory specific data;
receiving the accessory specific data (406) from each of the one or more of the one or more medical accessories once the communicative connection is established by a reader device (116, 40) of the gas analyzer;
for each of the one or more of the one or more medical accessories, determining compatibility with the gas analyzer based on the accessory specific data (408);
for each of the one or more of the one or more medical accessories, determining a remaining useful lifetime (410);
in response to each of the one or more of the one or more medical accessories being compatible with the gas analyzer, determining one or more operational parameters of the gas analyzer based on the accessory specific data (412, 900); and
in response to one or more of the one or more medical accessories being incompatible with the gas analyzer, displaying a first notification of incompatibility to a user via a user interface of the gas analyzer (512).

11. The method of claim 10, wherein the communicative connection between the one or more of the one or more medical accessories and the gas analyzer is established when the memory device of the one or more of the one or more medical accessories is within range of the reader device of the gas analyzer.

12. The method of claim 10, wherein determining the remaining useful lifetime comprises one or more of deploying a machine learning model and a mathematical algorithm (714).

13. The method of claim 10, wherein the remaining useful lifetime for a first accessory of the one or more medical accessories is determined based on 1) accessory specific data of the first accessory, wherein the accessory specific data of the first accessory comprises physical properties, functional properties, operational properties, and usage histories of the first accessory (706); 2) properties of the gas analyzer, wherein the properties of the gas analyzer comprise physical properties, functional properties, and a selected operational mode (708); 3) properties of other connected accessories of the one or more medical accessories (710); and 4) one or more values measured during use (712), wherein the one or more values comprise one or more of differential pressure, flow resistance, and flow rate.

14. The method of claim 10, wherein, for a given accessory of the one or more medical accessories, the accessory specific data comprises one or more of physical properties of the given accessory, a commissioning date, history of previous hosts, history of occlusion and nebulization events, bacterial and viral filtration efficiency, expected model of a compatible gas analyzer, available sampling flow rates, expected lifetime, expected differential pressure, an identifier, a batch, and orientation history (406).

15. The method of claim 10, further comprising monitoring accessory operation parameters for the one or more of the one or more medical accessories connected to the gas analyzer for detection of abnormal operation states (808).
